# EUROPEAN PATENT SPECIFICATION

(11) **EP 2 891 477 B1**
(45) Date of publication and mention of the grant of the patent: **26.04.2017**
(21) Application number: 13834035.1
(22) Date of filing: 22.08.2013
(51) Int. Cl.: A61F 9/007, B21D 41/04, B23K 26/20

(54) **Method of manufacturing vitreous body surgical probe**
Verfahren zur Herstellung einer chirurgischen Glaskörpersonde
Procédé de fabrication d'une sonde chirurgicale à corps vitreux

(30) Priority: 28.08.2012 JP 2012187207
(43) Date of publication of application: 08.07.2015
(73) Proprietor: MANI, INC., Utsunomiya-shi, Tochigi 321-3231 (JP)
(72) Inventor: MURAKAMI, Etsuo, Utsunomiya-shi Tochigi 321-3231 (JP)
(74) Representative: Isarpatent
(86) International application number: PCT/JP2013/072348
(87) International publication number: WO 2014/034506

(56) References cited:
- AT-U1- 6 938
- JP-A- H0 219 151
- JP-A- H06 262 283
- JP-A- 2003 509 181
- JP-A- 2009 511 169
- JP-U- H0 314 019
- NL-C2- 1 034 654
- US-A- 5 487 725
- US-A- 5 676 012
- US-A1- 2004 181 926
- US-A1- 2010 042 125

## Description

### [Technical Field]

The present invention relates to a vitreous body surgical probe used in eye operations and to a manufacturing method of the same.

### [Background Art]

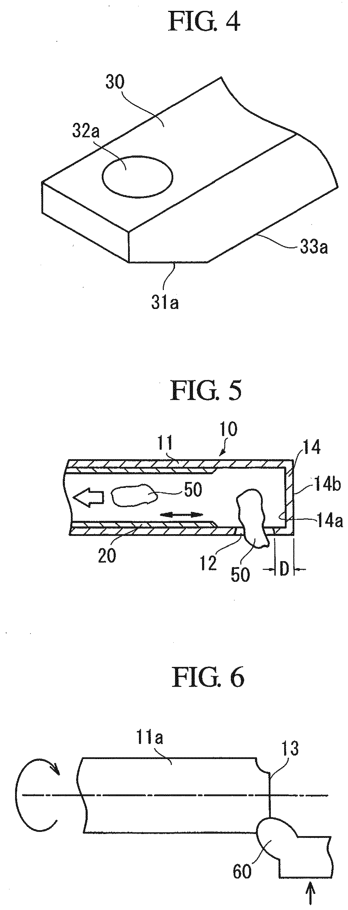

A vitreous body surgical probe used in eye operations is used for cutting/removing the gelatinous vitreous body in the eye and a proliferation membrane on the retina formed by degeneration of the vitreous body. FIG. 5 shows a cross-sectional view of a vitreous body surgical probe.

A vitreous body surgical probe 10 has a probe body 11 having a shape, in which one end of a tube is sealed with a distal part 14, and a cutting member 20, which is slidable along an axial direction while it is in contact with the inner surface of the probe body 11. An opening 12 is provided in a lateral surface in the vicinity of a distal end of the probe body 11, and a vitreous body, etc. 50 are suctioned therefrom. This process is configured so that the cutting member 20 slides, and the vitreous body, etc. 50 are cut when the distal end of the cutting member 20 passes the opening 12, and the vitreous body, etc. 50 cut into a small piece(s) is suctioned and collected to the rear side (leftward direction in FIG 5) of the probe 10.

In the vitreous body surgical probe 10, the distance D from the distal part 14 to the opening 12 is desired to be short. This is for a reason that the opening 12 has to be brought close to the retina as close as possible since the vitreous body, etc. 50 are close to the retina or in order to cut the proliferation membrane in the vicinity of the retina. A distal outer surface 14b is desired to be a flat surface without protrusions, etc. so that the probe body 11 does not touch and damage the retina.

A distal inner surface 14a is also desired to be a flat surface. This is for a reason that, if the distal inner surface 14a is not a flat surface, it becomes difficult to bring the distal end of the cutting member 20 close to the distal part 14 of the probe body 11 when the cutting member 20 slides, and, because of that, the distance D from the distal part 14 of the opening 12 has to be long.

As a method of forming the distal part 14 of the probe body 11 like this, Patent Literature 1 discloses a plastic working method of squeezing and processing a distal part of a tube, which is to serve as the probe body 11. FIG. 6 shows a drawing explaining such a plastic working method of a distal surface. Hereinafter, a tube having a distal part which is subjected to plastic working will be referred to as a plastic-working shaped tube.

Patent Literature 1 discloses a method in which a plastic-working shaped tube 11a is rotated about a main axis, and, while a spherical protrusion 60 is pressed against a tube distal part 13 thereof, the spherical protrusion 60 is moved in the radial direction of the plastic-working shaped tube 11a. As a result, the distal part of the plastic-working shaped tube 11a is plastically deformed gradually toward the inner side, and a distal part is finally formed. In such a plastic working method by squeezing, working is easy, and the distal outer surface approximately becomes a flat surface, but burrs are easily formed on the distal inner surface.

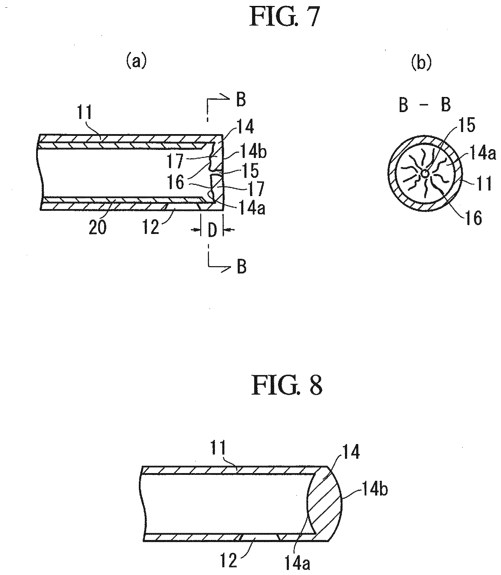

FIG. 7 is a drawing showing an end of the probe body, in which the distal surface is formed by the plastic working of FIG. 6; wherein, (a) is a cross-sectional view, and (b) is a cross-sectional view of a line B-B. The drawings show, as an example, a state in which the distal outer surface 14b is approximately formed into a flat surface, and burrs 16 are generated on the distal inner surface 14a. The structure of the distal part 14, which has undergone plastic working, has become a structure part 17 showing the flows of the plastic working.

In this plastic working method, working is carried out while squeezing is carried out from the outer side of the plastic-working shaped tube 11 a; therefore, a hole 15 may remain at the center of the distal part 14. If there is the hole 15 like this, a trouble such as suction of the retina therefrom upon use may occur. The hole 15 does not remain depending on plastic working; however, even if the hole 15 is closed without remaining, the center position thereof is not completely bonded.

The cutting member 20 slides in the axial direction while it is in contact with the inner surface of the probe body 11; wherein, if the distal end of the cutting member 20 collides with the distal inner surface 14a of the probe body 11, the distal end of the cutting member 20 may be chipped, and a trouble may occur. Thus, the cutting member 20 has to slide within a range in which the cutting member does not collide with the distal inner surface 14a of the probe body 11. Therefore, if the burrs 16 are formed on the distal inner surface 14a, the movable range of the cutting member 20 does not reach the vicinity of the distal part 14 of the probe body 11. Therefore, the distance D from the distal part 14 to the opening 12 has to be long.

As another working method of the distal part of the probe body, there is a method in which a distal part is formed by energy beam irradiation of laser or the like. FIG. 8 shows a cross-sectional view of an end of a probe body of a case in which a distal part is formed only by energy beam irradiation.

In order to form the distal part 14 only by energy beam irradiation, a wide range of the distal part of the tube has to be melted by energy beam irradiation. Therefore, the distal inner surface 14a of the probe body 11 bulges roundly. As a result, like the case in which the burrs are formed, the movable range of the cutting member does not reach the vicinity of the distal part 14 of the probe body 11, and the position of the opening becomes distant from the distal part 14. Moreover, since the distal outer surface 14b also similarly bulges, there is also a problem that it is difficult to bring the opening close to the retina.

Patent Document 2 discloses a manufacturing method of a vitreous body surgical probe whereby a distal part of the probe is swaged into a flattened distal end, the distal end is trimmed by a cutting wheel, a remaining hole is closed by fusing and finally a flat surface is pressed.

### [Prior Art Documents]

### [Patent Documents]

Patent Document 1 : Japanese Unexamined Patent Publication No. 2009-511169
Patent Document 2: US 2004/0181926 A1

### [Disclosure of Invention]

### [Problems to be Solved by the Invention]

As described above, if the method of forming the distal part of the probe body is only by the conventional plastic working using squeezing, burrs may be formed on the distal inner surface, and a hole may remain in the distal part. In the case of only energy beam irradiation, the distal part bulges to the inner side and the outer side. It is difficult to appropriately remove the vitreous body, etc. in the vicinity of the retina by the vitreous body surgical probe manufactured by these working methods.

Therefore, in view of such circumstances, it is an object of the present invention to provide a vitreous body surgical probe that forms an inner surface and an outer surface of a probe distal part into flat surfaces and prevents a hole from remaining in the distal part.

### [Means of Solving the Problem]

The invention provides a manufacturing method of a vitreous body surgical probe including, after a distal part covering a plastic-working shaped tube is formed onto a flat surface by subjecting one end of the plastic-working shaped tube serving as a probe body to plastic working, the distal part may be sealed by irradiating the distal part with an energy beam. A preferred method is provided in claim 2.

### [Result of the Invention]

According to the present invention, a vitreous body surgical probe in which an outer surface and an inner surface of a distal part of a probe body are flat surfaces and the distal part is completely sealed by energy beam irradiation can be provided.

### [Brief Description of Drawings]

FIG. 1 shows drawings showing a distal part of a probe body, which has undergone energy beam irradiation after plastic working; wherein, (a) is a cross-sectional view, and (b) is a cross-sectional view of a line A-A.
FIG. 2 is a drawing showing a distal part of a probe body, in which the distal part is an inclined surface.
FIG. 3 shows drawings explaining an example of plastic working steps; wherein, (a) shows a working step by a slope, (b) shows a working step by a spherical surface, and (c) shows a working step by a perpendicular step.
FIG. 4 is a distal-surface forming jig, which is integrated into one and is used in plastic working.
FIG. 5 is a cross-sectional view of a vitreous body surgical probe.
FIG. 6 is a drawing explaining a conventional plastic working method of a distal surface.
FIG. 7 shows drawings showing a distal part of a probe body, wherein a distal surface is formed by the plastic working of FIG. 4; wherein, (a) shows a cross-sectional view, and (b) shows a cross-sectional view of a line B-B.
FIG. 8 is a cross-sectional view of an end of a probe body of a case in which a distal part is formed only by energy beam irradiation.

### [Best Mode for Carrying Out the Invention]

Hereinafter, an embodiment of the present invention will be explained with reference to accompanying drawings.

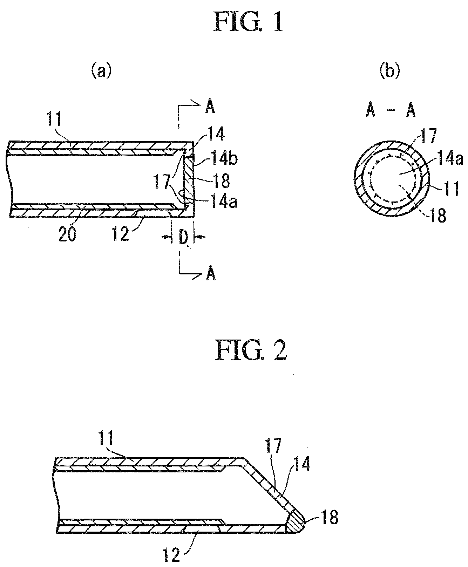

FIG. 1 shows drawings showing a distal part of a probe body, which has undergone energy beam irradiation after plastic working; wherein, (a) shows a cross-sectional view, and (b) shows a cross-sectional view of a line A-A.

A vitreous body surgical probe is manufactured by subjecting one end of a plastic-working shaped tube, which is to serve as the probe body, to plastic working to form the distal part covering the plastic-working shaped tube and then radiating an energy beam to seal the distal part. In the above described plastic working method shown in FIG. 6, the distal part mostly becomes a flat surface; however, the burrs 16 and the hole 15 are generated in some cases. Therefore, for example after the state of FIG. 7 (a) is obtained by plastic working, the distal part 14 is subjected to energy beam irradiation, thereby melting part of the distal part 14 and sealing the hole 15.

The energy beam irradiation carried out after the plastic working is preferred to be spot-manner irradiation at a temperature that melts metal. This is for a reason that a main object thereof is to cover the hole formed at the distal part. More specifically, this is for avoiding a situation that, if the temperature of the energy beam is increased more than needed or if irradiation time becomes long, a large amount of the distal part 14 is melted, and the distal part 14 bulges in inward/outward directions as shown in FIG. 8.

When energy beam irradiation is carried out from a distal outer surface 14b, the burrs 16 on a distal inner surface 14a can be melted, and the distal inner surface 14a can be further planarized as shown in the cross-sectional view of the line A-A. Thus, when energy beam irradiation is carried out, planarization of both of the distal outer surface 14b and the inner surface 14a can be ensured, and the hole 15 formed at the distal part can be sealed.

In a case in which both of such plastic working and energy beam irradiation is carried out, at first, when the plastic working is carried out, the metal structure of the distal part 14 becomes a state in which a structure part 17 showing flows of the plastic working in whole is formed. Then, since energy beam irradiation is carried out thereafter, the part melted by the irradiation becomes a granular structure 18 when solidified. In other words, the metal structure of the distal part 14 has the structure part 17 showing the flows of the plastic working and the granular structure 18. In this process, it is also possible to melt/solidify the whole structure part 17, which is formed at the distal part 14 by the plastic working, by energy beam irradiation and form it into the granular structure 18. In this case, most of the metal structure of the distal part 14 becomes the granular structure 18; however, also in this case, the structure part 17 formed by the plastic working and the granular structure 18 formed by melting/solidifying are assumed to be present in the probe body 11.

FIG. 2 is a drawing showing the distal part of a probe body in which the distal part is an inclined surface. When plastic working is carried out from one direction without rotating the plastic-working shaped tube, the distal part 14 can be formed into the inclined surface as shown in FIG. 2. When the distal part 14 is formed into an inclined surface in this manner, the probe body 11 can be brought further closer to the retina in some cases, and, by virtue of that, suction of the vitreous body, etc. from an opening 12 is facilitated. The angle of the inclined surface is determined by plastic working and is not particularly limited since a desired angle may be determined depending on the use thereof.

In the case in which the inclined surface is formed at the distal part 14, at the point when the plastic working is finished, the plastic-working part becomes the structure part 17 showing the flows of plastic working, and a hole remains at the distal end of the inclined surface. Therefore, the hole can be covered by forming the granular structure 18 by subjecting the distal end of the inclined surface to energy beam irradiation.

Next, an example of plastic working of forming a distal surface will be explained. In this embodiment, plastic working is carried out separately in three stages by using three types of jigs. When this method is used, the distal outer surface can be further planarized, and the hole remaining on the distal surface can be downsized or closed.

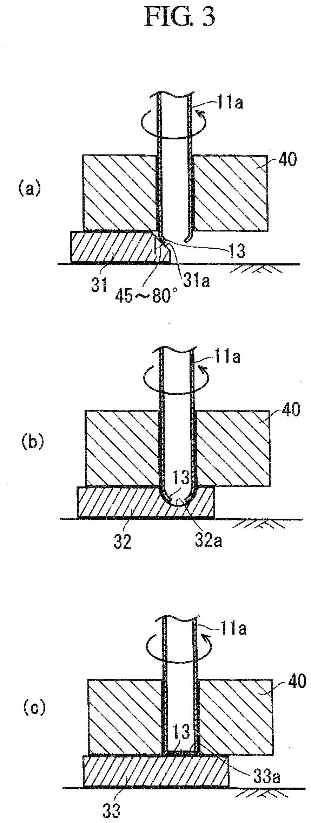

FIG. 3 shows drawings explaining an example of plastic working steps; wherein (a) shows a working step by a slope, (b) shows a working step by a spherical surface, and (c) shows a working step by a perpendicular surface. First, a holding jig 40 is caused to abut a lateral surface of a plastic-working shaped tube 11 a, which is to serve as a probe body, thereby limiting movement of the tube 11a toward a lateral side, and, at the same time, only a distal part 13 of the tube 11a is installed so as to project from the jig 40. The holding jig 40 may be a jig in which a hole for inserting the plastic-working shaped tube 11a is bored. Then, the tube 11a is rotated about a main axis. A drilling machine may be used for rotating the tube 11 a.

In FIG. 3 (a), a slope 31a is pressed against the distal part 13 of the tube 11a from a lateral side by using a first distal-surface forming jig 31 having the slope 31a, which is inclined with respect to the main-axis direction of the plastic-working shaped tube 11a. However, in this case, attention has to be paid so as not to excessively curve the distal part 13 of the tube 11a. If it is excessively curved, the distal part 13 is fractured, or burrs are easily generated; therefore, the distal part 13 is only required to be curved a little toward the inner side of the tube 1a. The angle of the slope 31a is about 45° to 80° with respect to the main-axis direction.

Next, in FIG. 3 (b), the distal part 13 of the plastic-working shaped tube 11a is pressed against a concave surface 32a by using a second distal-surface forming jig 32 having the concave surface 32a. Since the distal part 13 of the tube 11a has been brought into a state that it is curved a little toward the inner side by the step shown in FIG. 3 (a), the distal part 13 is further curved toward the inner side to process the distal part 13 into a round shape. The shape of the concave surface 32a is only required to smoothly curve the distal part 13 further toward the inner side and may be, for example, part of a spherical surface such as a semispherical surface.

Next, in FIG. 3 (c), the distal part 13 of the tube 11a is pressed against a perpendicular surface 33a by using a third distal-surface forming jig 33 having the surface 33a which is perpendicular to the main-axis direction of the plastic-working shaped tube 11a. After the step shown in FIG. 3 (b), the tube 11a is once pulled up, the second distal-surface forming jig 32 is replaced by the third distal-surface forming jig 33, and the distal part 13 is pressed against that while the tube 11a is rotated. As a result, the distal outer surface is formed into a flat surface.

In the plastic working method as described above, the distal part is deformed through the stages compared with the conventional plastic working; therefore, the distal part is less frequently broken upon plastic working due to buckling, etc., and the distal part can be planarized. In the case of this plastic working method, deformation is gradually carried out from the distal end of the tube, and it is once processed to be round; therefore, it is easy to control the deformation amount so that no hole remains at the center of the distal surface.

However, even in such a case, the hole remaining in the distal surface is closed, but is not bonded. Therefore, when energy beam irradiation is further carried out after the plastic working is carried out, the hole remaining in the distal surface can be closed and completely sealed.

As described above, the plastic working carried out before the energy beam irradiation may be that by conventional squeezing. However, when such plastic working of the three stages is carried out, the distal part can be reliably formed into a flat surface, and an excellent product can be always provided.

The jigs used in the plastic working of the three stages can be integrated into one jig. FIG. 4 shows a distal-surface forming jig in which the jigs used in the plastic working are integrated into one.

This distal-surface forming jig 30 has a concave surface 32a on an opposite surface of a slope 31a. An appropriate place can be used as the perpendicular surface 33a. Therefore, if there is this single distal-surface forming jig 30, the three jigs are not required to be prepared since required surfaces can be used depending on the respective steps, workability is improved.

### [Description of Reference Numerals]

- 10: (vitreous body surgical) Probe
- 11: Probe body
- 11a: (plastic-working shaped) Tube
- 12: Opening
- 13: Tube distal part
- 14: Distal part
- 14a: Distal inner surface
- 14b: Distal outer surface
- 15: Hole
- 16: Burr
- 17: Structure part showing flows of plastic working
- 18: Granular structure
- 20: Cutting member
- 30: End forming jig
- 31: First end forming jig
- 31a: Slope
- 32: Second end forming jig
- 32a: Concave surface
- 33: Third end forming jig
- 33a: Horizontal surface
- 40: Holding jig
- 50: Vitreous body

## Claims

1. A manufacturing method of a vitreous body surgical probe
**characterized by**,
after a distal part (14) covering a plastic-working shaped tube (11a) is formed into a flat surface by subjecting one end of the plastic-working shaped tube (11a) serving as a probe body (11) to plastic working, sealing the distal part (14) by irradiating the distal part with an energy beam.

2. The manufacturing method of the vitreous body surgical probe (10) according to claim 1, wherein
the plastic working is carried out from one direction without rotating the plastic-working shaped tube (11a), and
the distal part (14) is processed into an inclined surface having a desired angle.

## Patentansprüche

1. Verfahren zur Herstellung einer chirurgischen Glaskörpersonde, **gekennzeichnet durch**:
nachdem ein distaler Teil (14), der ein **durch** plastisches Umformen geformtes Rohr (11a) bedeckt, zu einer flachen Oberfläche geformt wurde, indem ein Ende des **durch** plastisches Umformen geformten Rohres (11a), das als Sondenkörper (11) dient, einem plastischen Umformen unterzogen wurde, Abdichten des distalen Teils (14) **durch** Bestrahlung des distalen Teils mit einem Energiestrahl.

2. Herstellungsverfahren der chirurgischen Glaskörpersonde (10) nach Anspruch 1, wobei
das plastische Umformen von einer Richtung ausgeführt wird, ohne das durch plastisches Umformen geformte Rohr (11a) zu drehen, und
der distale Teil (14) zu einer geneigten Oberfläche mit einem gewünschten Winkel verarbeitet wird.

## Revendications

1. Procédé de fabrication d'une sonde chirurgicale à corps vitreux, **caractérisée par** l'étape consistant à :
après qu'une partie distale (14) recouvrant un tube façonné par déformation plastique (11a) a été façonnée en une surface plane par façonnage par déformation plastique d'une extrémité du tube façonné par déformation plastique (11a) servant de corps de sonde (11), sceller la partie distale (14) en exposant la partie distale à un faisceau d'énergie.

2. Procédé de fabrication de la sonde chirurgicale à corps vitreux (10), selon la revendication 1, dans lequel :
le façonnage par déformation plastique est effectué depuis une direction, sans rotation du tube formé par façonnage par déformation plastique (11a), et
la partie distale (14) est transformée en une surface inclinée formant un angle souhaité.
